# EUROPEAN PATENT SPECIFICATION

(11) **EP 3 665 147 B1**
(45) Date of publication and mention of the grant of the patent: **19.10.2022**
(21) Application number: 18740904.0
(22) Date of filing: 20.06.2018
(51) Int. Cl.: C07C 51/00, C07C 51/44, C07D 307/50

(54) **PROCESS FOR PRODUCING LEVULINIC ACID**
VERFAHREN ZUR HERSTELLUNG VON LEVULINSÄURE
PROCÉDÉ DE PRODUCTION D'ACIDE LÉVULINIQUE

(30) Priority: 21.06.2017 IT 201700068744
(43) Date of publication of application: 17.06.2020
(73) Proprietor: Bio-On S.p.A., 40016 San Giorgio Di Piano (BO) (IT)
(72) Inventor: CONTI, Fabio, 40016 San Giorgio Di Piano (BO) (IT); BEGOTTI, Simone, 40016 San Giorgio Di Piano (BO) (IT); IPPOLITO, Feliciano, 40016 San Giorgio Di Piano (BO) (IT)
(74) Representative: Bottero, Carlo
(86) International application number: PCT/IB2018/054540
(87) International publication number: WO 2018/235012

(56) References cited:
- WO-A1-2014/087013
- WO-A1-2017/009217
- WO-A1-2017/009221

## Description

The present invention relates to a process for producing levulinic acid. More in particular, the present invention relates to a process for producing levulinic acid through heat treatment at acid pH of a carbohydrate and subsequent recovery of levulinic acid through a process that does not require the use of organic solvents.

Levulinic acid (also known as 4-oxopentanoic acid or γ-ketovaleric acid) is an organic product having formula:

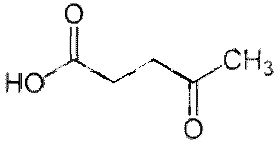

It can be widely used in the chemical industry, in particular as an intermediate for producing a wide variety of products such as resins, plasticizers, herbicides, solvents, additives for fuels, flavorings, pharmaceutical products, and the like.

Levulinic acid is usually produced through the treatment of carbohydrates at acid PH and at high temperature (up to 200°C), using a strong acid as a catalyst, such as hydrochloric acid or sulfuric acid.

For example, starting from sucrose, the reaction scheme that leads to the formation of levulinic acid is as follows (HMF = 2,5-(hydroxymethyl)furaldehyde or 2,5-(hydroxymethyl)furfural):
- sucrose (C₁₂H₂₂O₁₁) + H₂O --> glucose (C₆H₁₂O₆) + fructose (C₆H₁₂O₆) (I);
- fructose (C₆H₁₂O₆) --> HMF (C₆H₆O₃) + 3 H₂O (II)
- HMF (C₆H₆O₃) + 2 H₂O --> levulinic acid (C₅H₈O₃) + formic acid (CH₂O₂) (III)

   .

However, the yield of such process is actually rather low, mainly because of the formation of numerous reaction byproducts from which the levulinic acid must be separated through complex extraction and purification processes. As well as various low molecular weight byproducts, including formic acid, the heat treatment at acid pH of carbohydrates in pushed conditions leads to the formation of humins, which are high molecular weight products deriving from condensation reactions. Humins generally separate in the form of solids, usually dark colored, which cause numerous problems during the levulinic acid recovery process.

The recovery of levulinic acid and other products of interest, such as HMF, is generally performed through extraction with an appropriate organic solvent, selected for example from: ethyl acetate, methyl isobutyl ketone (MIBK), acetophenone, cyclohexanone, phenols and the like. On this point see for example patent application US 2006/0142599.

Patent application WO 98/19986 describes a process for producing levulinic acid that comprises the treatment of a cellulose or hemicellulose based biomass with a concentrated acid solution so as to form a gel. The gel is then diluted and heated to 80-100°C. After the separation of the liquid phase from the solid phase, the latter is treated again with a concentrated acid to 80-100°C. From such second treatment a liquid phase and a solid phase are obtained, and after being combined with the liquid phase obtained from the first separation, the liquid phase is hydrolyzed again in an acid environment at 40-240°C. The recovery of the levulinic acid is performed through passage of the reaction mixture through a chromatography column, in particular a multiple chromatography column system known as "simulated moving bed chromatography". This is a very slow recovery process that requires the use of complex and expensive apparatuses.

Patent application WO 2013/034763 describes a process for recovering levulinic acid or other useful products deriving from the acid hydrolysis of carbohydrates, wherein the reaction product is subjected to membrane separation in the presence of an organic solvent, using a nanofiltration membrane, which is impermeable to the molecules having a molecular weight of 100 kDa or more. In another embodiment, the recovery process of levulinic acid envisages a first reaction product distillation step, so as to obtain a distillate and a distillation residue, which is then subjected to separation through a nanofiltration membrane as described above.

WO 2017/009217 relates to a levulinic acid composition comprising levulinic acid, formic acid and angelica lactone, and to a process for isolating such composition.

WO 2014/087013 relates to a process for isolating levulinic acid from a raw composition, which is preferably obtained from acid hydrolysis of glucose or fructose.

WO 2017/009221 relates to a process for the isolation of levulinc acid obtained by acid catalyzed hydrolysis of a C6-carbohydrate-containing feedstock.

The Application set out to solve the problem of producing levulinic acid through a process with reduced environmental impact, without the use of organic solvents, that guarantees satisfactory process yields on an industrial scale, with a suitable degree of purity of the levulinic acid for its most common applications.

Such problem and others that will be better illustrated below have been solved through a process as defined in the present description and enclosed claims.

According to a first aspect, the present invention therefore relates to a process for producing levulinic acid, which comprises:
(a) subjecting a carbohydrate-based substrate to a treatment step, so as to obtain a reaction mixture comprising levulinic acid, 2,5-(hydroxymethyl)furfural (HMF), formic acid, water-insoluble humins and water-soluble humins;
(b) subjecting the reaction mixture to a filtration step, so as to obtain a solid phase comprising water-insoluble humins and a liquid phase comprising levulinic acid, HMF, formic acid, water-soluble humins;
(c) heating the liquid phase obtained by step (b), so as to obtain a liquid-vapor mixture at a temperature from 60°C to 95°C and a pressure from 150 mbara to 350 mbara;
(d) subjecting the liquid-vapor mixture obtained by step (c) to fractional distillation, so as to obtain:
   a water-based head phase,
   an intermediate phase comprising formic acid, and
   a tail-end phase comprising levulinic acid, HMF, and water-soluble humins;
(e) subjecting the tail-end phase obtained by step (d) to a separation process by a thin film evaporator at a temperature from 150°C to 220°C and a pressure from 100 mbara to 350 mbara, so as to obtain a gaseous phase comprising levulinic acid and HMF, and a liquid phase comprising water-soluble humins;
(f) subjecting the gaseous phase comprising levulinic acid and HMF to fractional distillation so as to separate the levulinic acid from HMF;
wherein the step (a) of treating is performed on a substrate containing sucrose and comprises the following sub-steps:
(a1) a first sub-step of acid hydrolysis of the sucrose for obtaining a mixture of glucose and fructose;
(a2) a second sub-step of separating the fructose from the glucose;
(a3) a third sub-step of treating the fructose, substantially free from glucose, in aqueous environment at acid pH and at a temperature from 120°C to 180°C, so as to obtain the reaction mixture comprising levulinic acid, 2,5-(hydroxymethyl)furfural (HMF), formic acid, water-insoluble humins and water-soluble humins.

Within the scope of the present description and appended claims, the pressure values are indicated, unless stated otherwise, as absolute pressure (mbara).

The process according to the present invention allows to produce levulinic acid and other useful products with high yields (around 35-45%), relatively contained production costs, and especially with a low environmental impact, as is it a process with a relatively low energy consumption with respect to known processes, and that does not require the use of polluting solvents or reactants.

At the end of the process, levulinic acid is obtained, which is the main product and is in the form of aqueous solution, with a concentration preferably from 50% to 90% by weight, more preferably from 70% to 85% by weight.

HMF is also obtained, which may be exploited as such, being a product that has various applications, e.g. for producing thermosetting resins and polyesters (through its derivative, furandicarboxylic acid).

Alternatively, the HMF obtained from step (f) can be reintroduced into the initial substrate that is subjected to the acid pH treatment step (a), so as to increase the overall levulinic acid yield.

In relation to step (a) of treating a carbohydrate-based substrate in aqueous environment at acid pH at a temperature from 120°C to 200°C, this is preferably carried out at pH from 0 to 5, more preferably from 0.5 to 4. The temperature is preferably from 120°C to 170°C. For obtaining the acid pH a mineral acid can be used, selected for example from: sulfuric acid, hydrochloric acid, phosphoric acid, nitric acid, boric acid, hydrofluoric acid, hydrobromic acid, or a Lewis acid. Preferably, diluted sulfuric acid can be used, for example at a concentration from 1.5 to 3% by weight.

The acidification of the reaction environment can also be obtained using an ion exchange resin containing acid groups, in particular sulfonic groups. A wide range of resins of this type is available commercially with the trademark Amberlite^{™} (Sigma Aldrich) (see for example patent US 2 738 367). Alternatively, it is possible to use a fluorinated ion exchange resin containing sulfonic groups, such as those known with the trademark Nafion^{™} (Du Pont). The use of an ion exchange resin also allows easier separation of the catalyst, which is solid, after the completion of the hydrolysis, with a lower environmental impact as the resin can be easily regenerated and reused.

Preferably step (a) is carried out at a pressure from 0.5 bar to 50 bar, more preferably from 1 bar to 20 bar. The reaction time can vary within large limits, mainly as a function of the substrate and acid used, as well as naturally the reaction conditions. In general, the reaction time ranges from 60 sec to 18 hours, more preferably from 60 min to 10 hours.

The carbohydrate-based substrate containing sucrose can be selected from a wide range of products, both substantially pure products, and mixtures of products, in general of natural origin, such as products deriving from the industrial processing of plants such as, for example, juices, molasses, pulps deriving from the processing of sugar beet or sugar cane.

The step (a) of treating in aqueous environment is performed on a substrate containing sucrose and comprises the following sub-steps:
(a1) a first sub-step of acid hydrolysis of sucrose for obtaining a mixture of glucose and fructose;
(a2) a second sub-step of separating the fructose from the glucose;
(a3) a third sub-step of treating the fructose, substantially free from glucose, in aqueous environment at acid pH and at a temperature from 120°C to 180°C, so as to obtain a reaction mixture comprising levulinic acid, 2,5-(hydroxymethyl)furfural (HMF), formic acid, water-insoluble humins and water-soluble humins.

The realization of the sub-step (a3) on fructose substantially free from glucose allows the production of humins to be considerably reduced, with a significant increase in the process yield and a simplification of the subsequent steps of separating the humins from the other reaction products.

In relation to the first sub-step (a1), it is preferably realized at a pH from 2 to 4. Preferably the reaction temperature is from 120°C to 150°C. Preferably, the reaction time is from 60 sec to 700 sec.

Sub-step (a2) can be realized according to known techniques, e.g. through ion exchange resin chromatography, in particular sulfonated resins with calcium ions as counterions.

In relation to the third sub-step (a3), it is preferably realized at a pH from 0.5 to 4. Preferably the reaction temperature is from 120°C to 170°C. Preferably, the reaction time is from 1 hour to 10 hours.

In relation to step (b) of filtrating the mixture of products obtained from step (a), it can be realized according to known techniques, in particular through membrane-orthogonal filtration. Filtration allows the insoluble humins to be separated as solid residue, while the liquid phase, comprising levulinic acid, HMF, formic acid, water-soluble humins, is sent to the subsequent steps. The insoluble humins can be disposed of for example through a biological purification process, possibly with the production of biogas, which can be used for the energy requirement of the system or for other uses.

The liquid phase obtained by the filtration step (b) is then heated, so as to obtain a liquid-vapor mixture at a temperature from 60°C to 95°C, preferably from 70°C to 90°C, and at a pressure from 150 mbara to 350 mbara, preferably from 200 mbara to 300 mbara.

The Applicant has noted that the heating of such liquid phase presents various problems, mainly connected with the fact that the heating of a considerable mass of liquid requires a long time, therefore the risk of degradation of the products present in the liquid increases, with the consequent lowering of the yield and the need to remove the degradation byproducts.

It is therefore particularly advantageous to perform such step of heating through a falling or rising film evaporator, in particular a falling film evaporator. These are known devices, in which the heating and transformation of liquid into vapor takes place inside a plurality of tubes, in turn heated by a fluid (e.g. vapor at low pressure), inside which the liquid flows in the form of film along the internal walls of the tubes themselves. In the case of a falling film evaporator, the film of liquid flows in the descending direction, thanks to the action of the force of gravity, while in the case of a rising film evaporator the film of liquid is pushed upwards by the vapor developed from boiling. In this way, the liquid is heated quickly, with rather reduced residence times at high temperatures with respect to conventional heating, and consequently a lower risk of degradation of the organic products present in the liquid itself.

In general, film evaporators allow very high heat exchange coefficients thanks to the speed of the liquid in contact with the tubes. Furthermore, the presence of a continuous vapor phase inside the tubes makes the heat exchanger suitable for operating at substantially uniform pressure as a result of the low load loss.

The liquid-vapor mixture at the outlet from the heating step (c) is then sent to the fractional distillation step (d), so as to obtain a water-based head phase, an intermediate phase comprising formic acid, and a tail-end phase comprising levulinic acid, HMF, and water-soluble humins. Fractional distillation (d) can be carried out according to known techniques, e.g. through a distillation column with structured filling.

At the head of the distillation column, water is substantially obtained which is in the form of vapor and, after condensation, can be reused in all the previous steps of the process, allowing a considerable reduction in water consumptions.

From the distillation column, an intermediate phase is also obtained, preferably in gaseous form, mainly comprising formic acid. The formic acid obtained in the gaseous phase is then subjected to condensation and sent to storage.

It is important to underline that the intermediate phase containing the formic acid is preferably in gaseous phase, and not in liquid phase, and is therefore taken at a point of the column, below the feed plate of the liquid-vapor mixture at the outlet from the heating step (c), where the mixture is still in the gaseous phase. In fact, if the formic acid were extracted from the column in liquid form, it would contain significant quantities of levulinic acid, which would then be separated from the formic acid with other means, with an inevitable reduction of the overall yield of the levulinic acid recovery process.

The tail-end phase, in liquid form, comprising levulinic acid, HMF and water-soluble humins is then sent to a separation process through a thin film evaporator at a temperature from 150°C to 220°C, preferably from 180°C to 210°C, and a pressure from 100 mbara to 350 mbara, preferably from 150 mbara to 300 mbara, so as to obtain a gaseous phase comprising levulinic acid and HMF, and a liquid phase comprising water-soluble humins.

Unlike a falling or rising film evaporator, the thin film evaporator comprises a single tube inside which the liquid to be treated flows along the internal wall of the tube itself. The film of liquid is distributed uniformly on the wall thanks to the action of a blade-rotor inserted inside the tube which, when placed in rotation, as well as distributing the liquid on the wall, creates a turbulent flow in the film itself which substantially improves the heat exchange. This type of evaporator allows the more volatile portion to be quickly separated from the less volatile portion, thanks to the agitation of the liquid in the form of film in controlled conditions. The evaporator preferably operates at reduced pressure to lower the separation temperature of the vapor phase from the liquid phase. The heating of the wall of the tube takes place for example through external coils inside which a heating fluid flows, e.g. water vapor.

The liquid phase comprising the water-soluble humins can be sent for disposal, e.g. through a biological purification process, possibly with the production of biogas, which can be the same used for the disposal of the water-insoluble humins as indicated above.

The vapor phase comprising levulinic acid and HMF is sent to the fractional distillation step (f), so as to separate the levulinic acid from HMF.

Fractional distillation (f) can be carried out according to known techniques, e.g. through a distillation column with suitable filling for operating at reduced pressures.

The cooling and condensation of the levulinic acid can be optionally integrated with production of vapor at low pressure, thus guaranteeing energy saving (in fact, the head vapors are typically between 140 and 180°C).

From the fractional distillation step a head phase is obtained substantially consisting of levulinic acid, and a tail-end phase essentially consisting of HMF. The degree of purity of such flows obviously depends on the separation conditions in the column. If necessary, according to the use that is intended to be made of these two products, they can be further purified according to known techniques.

The present invention will now be further illustrated through:

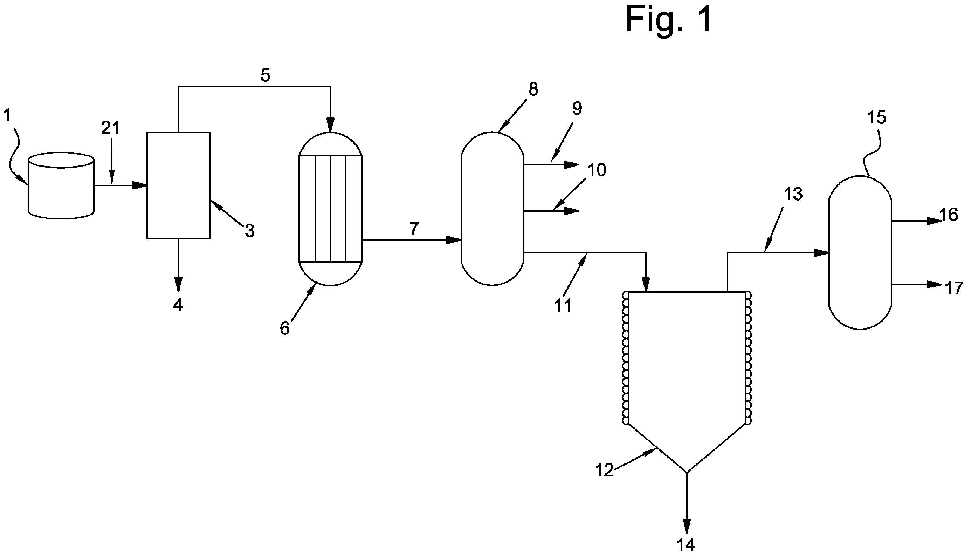

Figure 1, which is a schematic representation of a plant adapted to perform the claimed process.

With reference to Figure 1, the carbohydrate-based substrate is fed into a reactor (1), where it is treated in aqueous environment at acid pH at a temperature from 120°C to 200°C, so as to obtain a flow (2) at the outlet of the reaction mixture comprising levulinic acid, 2,5-(hydroxymethyl)furfural (HMF), formic acid, water-insoluble humins and water-soluble humins.

The flow (2) is fed to a filtration step, e.g. through a membrane-orthogonal filter (3) from which a solid residue (4) is obtained and a flow (5) of the liquid phase comprising levulinic acid, HMF, formic acid, water-soluble humins. The flow (5) of the liquid phase is sent to the heating step which is preferably realized in a falling film evaporator (6). The liquid-vapor mixture (7) obtained from the evaporator (6) is sent to a fractional distillation column (8), from which three phases are obtained: a head phase (9) essentially consisting of water; an intermediate phase (10) essentially consisting of formic acid, and a tail-end phase (11) essentially consisting of levulinic acid, HMF and water-soluble humins.

The tail-end phase (11) is then sent to a thin film evaporator (12), operating at a temperature from 150°C to 220°C and at a pressure from 100 mbara to 350 mbara. From the evaporator (12) a gaseous phase (13) is obtained, essentially consisting of levulinic acid and HMF, and a liquid phase (14) essentially consisting of water-soluble humins.

Finally, the gaseous phase (13) is sent to a fractional distillation column (15), from which two distinct flows are obtained: a head flow (16) essentially consisting of levulinic acid, and a tail-end flow (17) essentially consisting of HMF.

## Claims

1. Process for producing levulinic acid, comprising:
(a) subjecting a carbohydrate-based substrate to a treatment step, so as to obtain a reaction mixture comprising levulinic acid, 2,5-(hydroxymethyl)furfural (HMF), formic acid, water-insoluble humins and water soluble humins;
(b) subjecting the reaction mixture to a filtration step, so as to obtain a solid phase comprising water-insoluble humins and a liquid phase comprising levulinic acid, HMF, formic acid, water-soluble humins;
(c) heating the liquid phase obtained by step (b), so as to obtain a liquid-vapor mixture at a temperature from 60°C to 95°C and a pressure from 150 mbara to 350 mbara;
(d) subjecting the liquid-vapor mixture obtained by step (c) to fractional distillation, so as to obtain:
a water-based head phase,
an intermediate phase comprising formic acid, and
a tail-end phase comprising levulinic acid, HMF, and water-soluble humins;
(e) subjecting the tail-end phase obtained by step (d) to a separation process by a thin film evaporator at a temperature from 150°C to 220°C and a pressure from 100 mbara to 350 mbara, so as to obtain a gaseous phase comprising levulinic acid and HMF, and a liquid phase comprising water-soluble humins;
(f) subjecting the gaseous phase comprising levulinic acid and HMF to fractional distillation so as to separate the levulinic acid from HMF;
wherein the step (a) of treating is performed on a substrate containing sucrose and comprises the following sub-steps:
(a1) a first sub-step of acid hydrolysis of the sucrose for obtaining a mixture of glucose and fructose;
(a2) a second sub-step of separating the fructose from the glucose;
(a3) a third sub-step of treating the fructose, substantially free from glucose, in aqueous environment at acid pH and at a temperature from 120°C to 180°C, so as to obtain the reaction mixture comprising levulinic acid, 2,5-(hydroxymethyl)furfural (HMF), formic acid, water-insoluble humins and water-soluble humins.

2. Process according to claim 1, wherein the step (a) of treating the substrate is carried out at a pH from 0 to 5, preferably from 0.5 to 4.

3. Process according to any one of the preceding claims, wherein the step (a) of treating is carried out at a pressure from 0.5 bar to 50 bar, preferably from 1 bar to 20 bar.

4. Process according to any one of the preceding claims, wherein the step (b) of filtrating is carried out by membrane-orthogonal filtration.

5. Process according to any one of the preceding claims, wherein the step (c) of heating is carried out by a falling or rising film evaporator, in particular a falling film evaporator.

6. Process according to any one of the preceding claims, wherein the intermediate phase comprising formic acid is in gaseous form.

7. Process according to any one of the preceding claims, wherein the thin film evaporator comprises a single tube inside which the tail-end phase to be treated flows along an internal wall of the tube itself, and the tail-end phase is distributed uniformly on said wall thanks to the action of a blade-rotor inserted inside the tube.

## Patentansprüche

1. Verfahren zur Herstellung von Lävulinsäure, umfassend:
(a) Unterziehen eines Substrats auf Kohlenhydratbasis einem Behandlungsschritt, um eine Reaktionsmischung zu erhalten, die Lävulinsäure, 2,5-(Hydroxymethyl)furfural (HMF), Ameisensäure, wasserunlösliche Huminstoffe und wasserlösliche Huminstoffe umfasst;
(b) Unterziehen der Reaktionsmischung einem Filtrationsschritt, um eine feste Phase, die wasserunlösliche Huminstoffe umfasst, und eine flüssige Phase, die Lävulinsäure, HMF, Ameisensäure, wasserlösliche Huminstoffe umfasst, zu erhalten;
(c) Erhitzen der durch Schritt (b) erhaltenen flüssigen Phase, um eine Flüssigkeits-Dampf-Mischung bei einer Temperatur von 60 °C bis 95 °C und einem Druck von 150 mbara bis 350 mbara zu erhalten;
(d) Unterziehen der durch Schritt (c) erhaltenen Flüssigkeits-Dampf-Mischung einer fraktionierten Destillation, um Folgendes zu erhalten:
eine wasserbasierte Kopfphase,
eine Zwischenphase, die Ameisensäure umfasst, und eine Tailend-Phase, die Lävulinsäure, HMF und wasserlösliche Huminstoffe umfasst;
(e) Unterziehen der durch Schritt (d) erhaltenen Tailend-Phase einem Trennverfahren durch einen Dünnschichtverdampfer bei einer Temperatur von 150°C bis 220°C und einem Druck von 100 mbara bis 350 mbara, um eine gasförmige Phase, die Lävulinsäure und HMF umfasst, und eine flüssige Phase, die wasserlösliche Huminstoffe umfasst, zu erhalten;
(f) Unterziehen der gasförmigen Phase, die Lävulinsäure und HMF umfasst, einer fraktionierten Destillation, um die Lävulinsäure vom HMF zu trennen;
wobei der Schritt (a) der Behandlung auf einem Saccharose enthaltenden Substrat durchgeführt wird und die folgenden Teilschritte umfasst:
(a1) einen ersten Teilschritt der sauren Hydrolyse der Saccharose, um eine Mischung aus Glukose und Fruktose zu erhalten;
(a2) einen zweiten Teilschritt zur Trennung der Fruktose von der Glukose;
(a3) einen dritten Teilschritt der Behandlung der Fruktose, die im Wesentlichen frei von Glukose ist, in wässriger Umgebung bei einem sauren pH-Wert und bei einer Temperatur von 120°C bis 180°C, um die Reaktionsmischung zu erhalten, die Lävulinsäure, 2, 5-(Hydroxymethyl)furfural (HMF), Ameisensäure, wasserunlösliche Huminstoffe und wasserlösliche Huminstoffe umfasst.

2. Verfahren nach Anspruch 1, wobei der Schritt (a) der Behandlung des Substrats bei einem pH-Wert von 0 bis 5, vorzugsweise von 0,5 bis 4, durchgeführt wird.

3. Verfahren nach einem der vorhergehenden Ansprüche, wobei der Schritt (a) der Behandlung bei einem Druck von 0,5 bar bis 50 bar, vorzugsweise von 1 bar bis 20 bar, durchgeführt wird.

4. Verfahren nach einem der vorhergehenden Ansprüche, wobei der Schritt (b) des Filtrierens durch membranorthogonale Filtration durchgeführt wird.

5. Verfahren nach einem der vorhergehenden Ansprüche, wobei der Schritt (c) des Erhitzens durch einen Fall- oder Steigfilmverdampfer, insbesondere einen Fallfilmverdampfer, durchgeführt wird.

6. Verfahren nach einem der vorhergehenden Ansprüche, wobei die Ameisensäure umfassende Zwischenphase in gasförmiger Form vorliegt.

7. Verfahren nach einem der vorhergehenden Ansprüche, wobei der Dünnschichtverdampfer ein einzelnes Rohr umfasst, in dessen Innerem die zu behandelnde Tailend-Phase entlang einer Innenwand des Rohres selbst fließt, und die Tailend-Phase ist auf dieser Wand dank der Wirkung eines in das Rohr eingesetzten Blattrotors gleichmäßig verteilt.

## Revendications

1. Procédé de production d'acide lévulinique, comprenant:
(a) la soumission d'un substrat à base de glucides à une étape de traitement, de manière à obtenir un mélange réactionnel comprenant de l'acide lévulinique, du 2,5-(hydroxyméthyl)furfural (HMF), de l'acide formique, des humines insolubles dans l'eau et des humines solubles dans l'eau;
(b) la soumission du mélange réactionnel à une étape de filtration, de manière à obtenir une phase solide comprenant des humines insolubles dans l'eau et une phase liquide comprenant de l'acide lévulinique, du HMF, de l'acide formique, des humines solubles dans l'eau;
(c) le chauffage de la phase liquide obtenue à l'étape (b), de manière à obtenir un mélange liquide-vapeur à une température de 60 °C à 95 °C et à une pression de 150 mbar à 350 mbar;
(d) la soumission du mélange liquide-vapeur obtenu à l'étape (c) à une distillation fractionnée, de manière à obtenir:
une phase de début à base d'eau,
une phase intermédiaire comprenant de l'acide formique, et une phase finale comprenant de l'acide lévulinique, du HMF et des humines solubles dans l'eau;
(e) la soumission de la phase finale obtenue à l'étape (d) à un procédé de séparation par un évaporateur à couche mince à une température de 150 °C à 220 °C et à une pression de 100 mbar à 350 mbar, de manière à obtenir une phase gazeuse comprenant de l'acide lévulinique et du HMF, et une phase liquide comprenant des humines solubles dans l'eau;
(f) la soumission de la phase gazeuse comprenant l'acide lévulinique et le HMF à une distillation fractionnée de manière à séparer l'acide lévulinique du HMF;
dans lequel l'étape (a) de traitement est réalisée sur un substrat contenant du saccharose et comprend les sous-étapes suivantes:
(a1) une première sous-étape d'hydrolyse acide du saccharose pour obtenir un mélange de glucose et de fructose;
(a2) une deuxième sous-étape de séparation du fructose du glucose;
(a3) une troisième sous-étape de traitement du fructose, sensiblement exempt de glucose, en milieu aqueux à pH acide et à une température de 120 °C à 180 °C, de manière à obtenir le mélange réactionnel comprenant de l'acide lévulinique, du 2, 5-(hydroxyméthyl)furfural (HMF), de l'acide formique, des humines insolubles dans l'eau et des humines solubles dans l'eau.

2. Procédé selon la revendication 1, dans lequel l'étape (a) de traitement du substrat est réalisée à un pH de 0 à 5, de préférence de 0,5 à 4.

3. Procédé selon l'une quelconque des revendications précédentes, dans lequel l'étape (a) de traitement est réalisée à une pression comprise entre 0,5 bar et 50 bar, de préférence entre 1 bar et 20 bar.

4. Procédé selon l'une quelconque des revendications précédentes, dans lequel l'étape (b) de filtration est mise en œuvre par filtration orthogonale sur membrane.

5. Procédé selon l'une quelconque des revendications précédentes, dans lequel l'étape (c) de chauffage est mise en œuvre par un évaporateur à film descendant ou ascendant, en particulier un évaporateur à film descendant.

6. Procédé selon l'une quelconque des revendications précédentes, dans lequel la phase intermédiaire comprenant l'acide formique est sous forme gazeuse.

7. Procédé selon l'une quelconque des revendications précédentes, dans lequel l'évaporateur à couche mince comprend un seul tube à l'intérieur duquel la phase finale à traiter s'écoule le long d'une paroi interne du tube lui-même, et la phase finale est distribuée uniformément sur ladite paroi grâce à l'action d'un rotor à pales inséré à l'intérieur du tube.
